# EUROPEAN PATENT SPECIFICATION

(11) **EP 3 744 367 B1**
(45) Date of publication and mention of the grant of the patent: **17.09.2025**
(21) Application number: 19205345.2
(22) Date of filing: 25.10.2019
(51) Int. Cl.: A61M 5/158, A61M 5/142, A61M 5/162, A61M 5/14, A61M 39/10

(54) **NEEDLE INFUSION SET FOR INSULIN INFUSION**
NADELINFUSIONSSET FÜR INSULININFUSION
ENSEMBLE DE PERFUSION D'AIGUILLE POUR PERFUSION D'INSULINE

(30) Priority: 29.05.2019 KR 20190063177
(43) Date of publication of application: 02.12.2020
(73) Proprietor: Sooil Development Co., Ltd., Seoul 06274 (KR)
(72) Inventor: CHOI, Soo Bong, Seoul (KR)
(74) Representative: Sander, Rolf

(56) References cited:
- EP-A1- 1 820 525
- EP-A1- 1 917 990
- WO-A1-2017/007968
- WO-A1-94/20160
- KR-B1- 101 802 110
- US-A1- 2012 143 136
- US-A1- 2016 303 314

## Description

### BACKGROUND OF THE INVENTION

### Field of the invention

The present disclosure relates to a needle infusion set for insulin infusion, and more particularly, to a needle infusion set for insulin infusion in which an infusion needle is stuck in a body and fixed to an attachment pad and then insulin is infused into the body, wherein one or at least two infusion needles are integrally coupled to each other.

### Related Art

Diabetes as a disease based on metabolism abnormality caused due to lack of insulin which is hormone released from pancreas is a disease in which a blood sugar level is excessively increased as compared with a normal person to cause various complications and when the disease is neglected, a patient falls into diabetic coma to die.

In the treatment of the diabetes, a diet that restricts a food intake amount of the patient, an appropriate exercise, and a method for actively infusing drugs or insulin may be selectively or complexly are used.

Among them, a method by an insulin infusion device (also referred to as an 'insulin pump') is a method that consecutively and automatically adjusts an amount of insulin and infuses the insulin according to an individual diabetic status. Accordingly, since the insulin infusion device serves as a kind of artificial pancreas organ, the patient may live like the normal person and a special diet is not required.

The insulin infusion device automatically supplies a predetermined insulin amount to an insulin infusion set by minutely driving a motor included therein.

The insulin infusion set is a device that infuses the insulin supplied from the insulin infusion device into the body by inserting a catheter into a body fat layer of the abdomen at all times.

As the prior art, an infusion set of International Publication No. WO 2007/092210 (International publication date: August 16, 2007) includes a base member 60, an inserter cap 64, and an infusion cap 54. Here, the base member 60 includes a cannula 52 which is projected from a lower surface 118 of the base member 60 and a port 62 on an upper surface 92 of the base member 60. The port 62 is configured to transfer a fluid to the cannula 52 and includes a partition 130 sealing the port 62 for undesired fluid transfer. The inserter cap 64 is configured to be mounted on the base member 60 and has a needle 66 configured to extend through the partition 130 and the cannula 52 at an assembled location. The infusion cap 54 includes a lumen 160 configured to receive a flexible tube 162 and includes a hard cannula 170 which is configured to be inserted through the partition 130 and configured to position the cannula 52 so as to transfer the fluid to and from the lumen 160 (see reference numeral 29 in the drawing).

The prior art is configured in such a manner that the base member 60 is attached to a skin of the patient, the needle 66 and the cannula 52 are inserted through the skin of the patient, the inserter cap 64 to which the needle 66 is attached is removed from the base member 60 and then the infusion cap 54 is coupled to the base member 60. In this case, when the infusion cap 54 is coupled, a thorny leg 94 of the infusion cap 54 is pressed and fastened to a rim 96 of the base member 60 and a release handle 100 of the infusion cap 54 is gripped and force is applied to the release handle 100 to be released from the base member 60.

Further, the inserter cap 64 to which the needle 66 is attached should not be reused and discarded after the cannula 52 is inserted into the skin and then removed
from the base member 60. In addition, since the cannula 52 is soft, the cannula 52 cannot but be manufactured so that a diameter is large, and as a result, when the cannula 52 is inserted into or removed from the subcutaneous fat, there is a problem in that scratches or pain in the skin are caused.

Further, the insulin infusion set in the related art, which includes the prior art, is not yet produced in Korea and all insulin infusion sets are imported from foreign countries and used, and as a result, there is a problem in that the insulin infusion sets are expensive. Accordingly, there is a situation in which localization of all insulin infusion sets which depend only on importation is urgently required.
KR 101 802 110 B1 discloses an insulin infusion set which comprises a catheter fixing member (10) and a tube pipe connecting member (30). In the catheter fixing member (10), a funnel-type insertion unit (60) having a catheter (70) connected to a bottom part thereof, a connecting member (50), and a sealing member (40) are arranged sequentially.
WO 2017 007 968 A1 discloses multi-medicament infusion systems for preventing the cross-channeling of medicaments.
US 2012/143136 A1 discloses an infusion set having a disposable inserter that can insert a needle at a controlled rate of speed to a depth to deliver insulin or other medicament to the upper 3 mm of skin surface.

### SUMMARY OF THE INVENTION

In order to solve the aforementioned problem, a technical object to be achieved by the present disclosure is to provide a needle infusion set for insulin infusion, in which infusion needles inserted into a body and supplying insulin are integrally coupled to each other.

Furthermore, another technical object to be achieved by the present disclosure is to provide a needle infusion set for insulin infusion, in which a size (31 to 33 gauges) of an infusion needle is formed to be thinner than that of the related art (27 to 29 gauges of a needle) and the needle flexibly moves with movement of the muscle, and as a result, there is almost no feeling that the needle is inserted and there is no pain at the time of inserting or releasing the needle into or from a skin.

Furthermore, yet another technical object to be achieved by the present disclosure is to provide a needle infusion set for insulin infusion, in which two or more multi-channel infusion needles are integrally configured and crumpling of a subcutaneous fat layer which frequently occurs due to dispersion of an insulin liquid and infusion of insulin may be reduced.

Furthermore, still yet another technical object to be achieved by the present disclosure is to provide a needle infusion set for insulin infusion, in which two or more multi-channel infusion needles are integrally configured and insulin is smoothly supplied through an infusion needle at another portion even though one portion of the infusion needle is blocked.

Furthermore, still yet another technical object to be achieved by the present disclosure is to provide a needle infusion set for insulin infusion in which a needle fixation member and a tube connection member are easily separated from and coupled to each other.

Furthermore, still yet another technical object to be achieved by the provide invention is to present a needle infusion set for insulin infusion in which an attachment pad is bonded to a lower surface of a needle fixation member and an infusion needle is inserted into a body and then the attachment pad is attached to the body.

An object to be solved by the present disclosure is not limited to the aforementioned effects, and other objects, which are not mentioned above, will be clearly appreciated by a person having ordinary skill in the art from the following description.

The subject matter of the present invention is defined in the appended independent claim. Embodiments are defined in the appended dependent claims. As a means for solving the technical problem, a needle infusion set for insulin infusion according to the present disclosure includes: a needle fixation member (10); and a tube connection member (30), in which the needle fixation member (10) is configured to include a body (11) in which the other side is configured in an elliptical shape and the body (11) has a rectangular shape in which one side has a smaller
width than the other side, insertion grooves (14) are formed on both lateral surfaces of the rectangle, a base (12) is formed to be flat with a step from the rectangular shape, a guide groove (13) is formed at one upper portion of the base (12) in a longitudinal direction, and fitting protrusions (17) are vertically formed on both lateral surfaces of the base (12), upper and lateral staking protrusions (15, 16) formed to protrude from a top surface and a lateral surface of one side of the body (11) and having upper and lateral insertion holes (18, 19) passing through an internal space therein, one or two or more infusion needle insertion holes (20, 24) formed to vertically penetrate from a lower surface in the internal space of the body (11), upper and lateral sealing members (40, 41) inserted into the upper and lateral insertion holes (18, 19) and sealing the internal space and then fixed by staking the upper and lateral staking protrusions (15, 16), and protection caps (23, 27) inserted into cap insertion grooves (22, 26) formed on the lower surface of the body (11) and protecting the infusion needles (21, 25).

The tube connection member (30) may be configured to include a body (31) in which a tube (36) supplying insulin is connected to one side of the body (31) and an infusion needle (37) inserted into the lateral sealing member (41) protrudes on the other side of the body (31), a hook lever (33) formed to have elastic force on both outer sides of the body (31) and having receiving grooves (33) to which a hook protrusion (35) and the fitting protrusion (17) are fastened at an outer side, and an insertion portion (32) formed between the hook lever (33) and the infusion needle (37) and inserted into the insertion groove (14).

The needle infusion set for insulin infusion is configured to simultaneously supply the insulin through a connection hole (28) connecting the infusion needle insertion holes (20, 24) to each other inside the body (11) when there are two or more infusion needle insertion holes (20, 24).

The needle fixation member (10) may be configured to further include an attachment pad (50) bonded to the lower surface of the body (11) attached to the body to fix the needle fixation member (10).

The infusion needles (21, 25) may have a diameter of 31 to 33 gauges.

According to the present disclosure, when insulin is habitually infused each time the insulin is infused, pain and inconvenience are caused and a skin is scratched, and as a result, there is a risk of infection, but an infusion needle coupled to a needle fixation member is inserted into a body and then attached with a pad and the insulin is infused into the body through the infusion needle, thereby removing inconvenience of infusing the insulin several times in order to infuse the insulin and the pain caused during an aggression process.

Furthermore, when clothes are changed or a bath is taken, if a tube connection member is separated from the needle fixation member attached to the body, a tube connected to a tube connection member and an insulin infusion device (insulin pump)
may be together separated, and as a result, the bath can be freely taken.

Furthermore, two or more infusion needles are integrally configured to reduce crumpling of a subcutaneous fat layer which frequently occurs in insulin infusion due to dispersion of an insulin liquid.

Furthermore, two or more infusion needles are constituted in parallel to smoothly supply the insulin through the infusion at another portion even though one portion of the infusion needle is blocked.

The effects of the present disclosure are not limited to the aforementioned effects, and other effects, which are not mentioned above, will be clearly appreciated by a person having ordinary skill in the art from the following description.

### BRIEF DESCRIPTION OF THE DRAWINGS

FIGS. 1 to 6 are diagrams illustrating a needle infusion set for insulin infusion according to a first preferred embodiment of the present disclosure.
FIGS. 1 and 2 are perspective views.
FIGS. 3 and 4 are exploded perspective views.
FIG. 5 is a perspective view in which a needle fixation member 10 and a tube connection member 30 are separated from each other.
FIG. 6 is a cross-sectional view illustrating an internal configuration.
FIGS. 7 to 13 are diagrams illustrating a needle infusion set for insulin infusion according to a second preferred embodiment of the present disclosure.

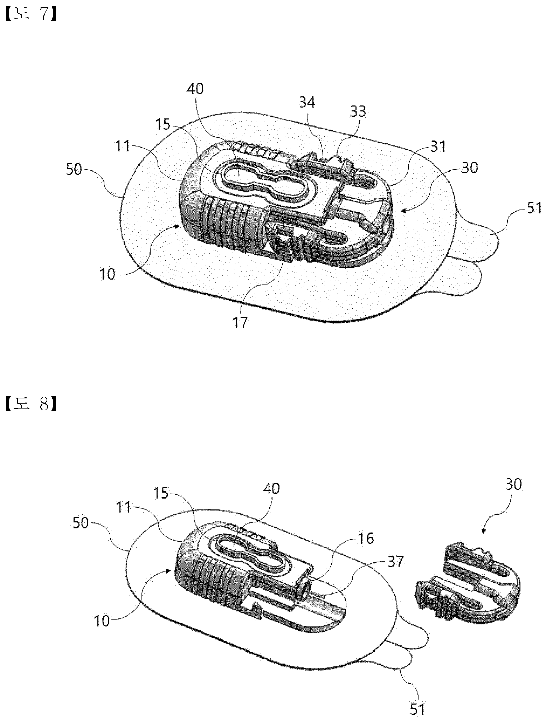

FIG. 7 is a perspective view.

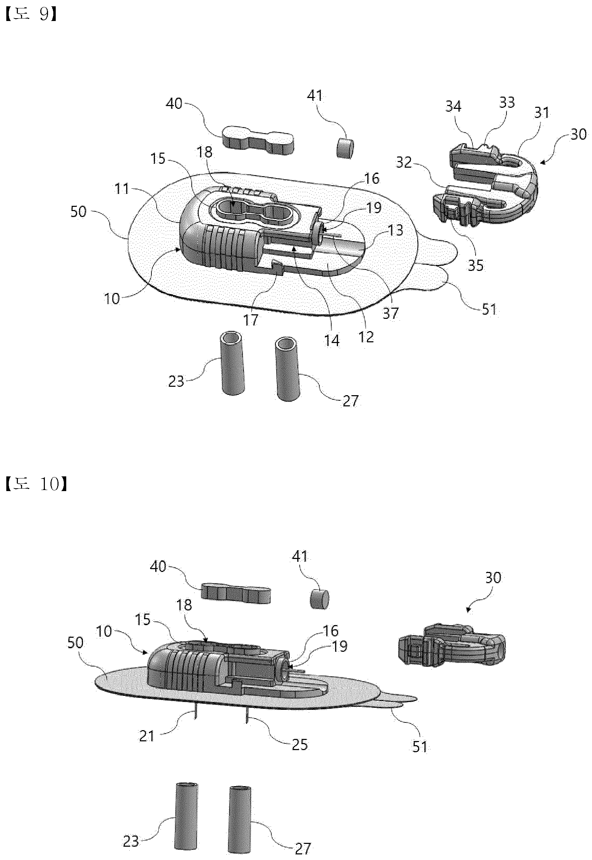

FIGS. 8 to 10 are exploded perspective views.

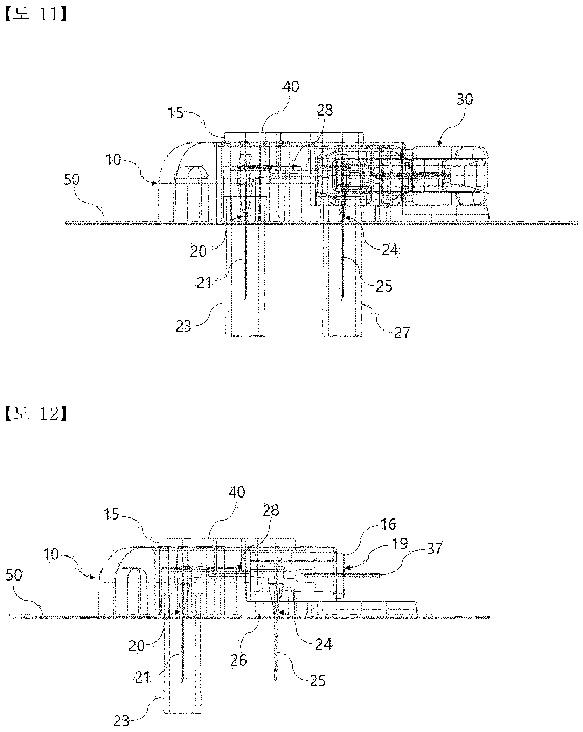

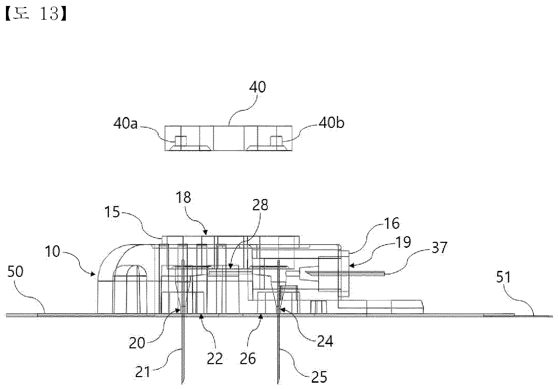

FIGS. 11 to 13 are cross-sectional views illustrating an internal configuration.

### DESCRIPTION OF EXEMPLARY EMBODIMENTS

Hereinafter, embodiments of the present disclosure will be described in detail so as to be easily implemented by those skilled in the art, with reference to the accompanying drawings. However, the present disclosure can be realized in various different forms, and is not limited to the exemplary embodiments described herein. In addition, in the drawings, in order to clearly describe the present disclosure, a part not related to the description is not omitted and like reference numerals designate like elements throughout the description of the present disclosure.

Hereinafter, detailed technical contents to be implemented by the present disclosure will be described in detail with reference to the accompanying drawings.

### First embodiment

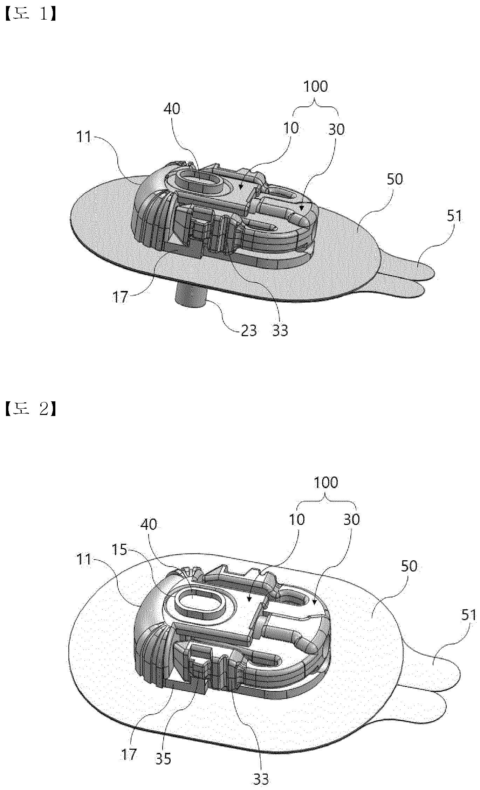

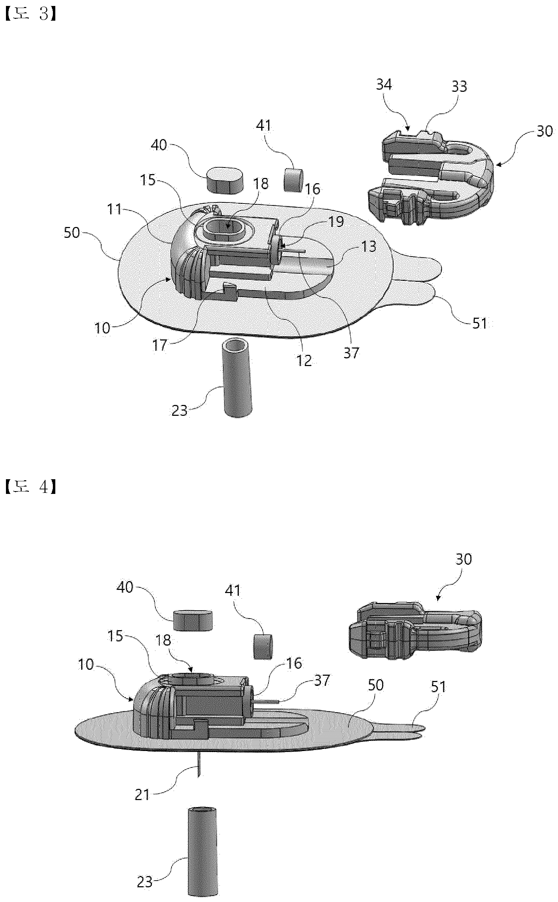

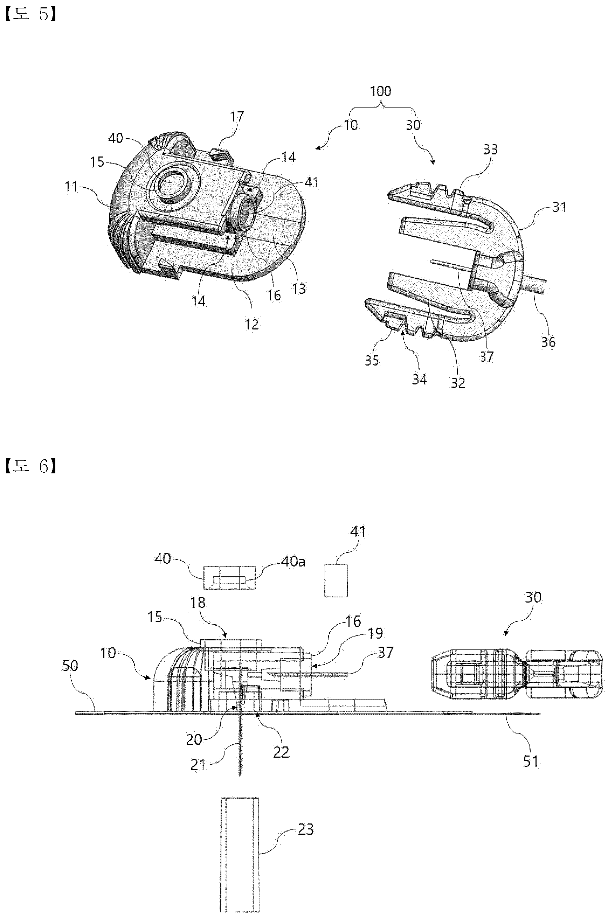

FIGS. 1 to 6 are diagrams illustrating a needle infusion set for insulin infusion according to a first preferred embodiment of the present disclosure; FIGS. 1 and 2 are perspective views, FIGS. 3 and 4 are exploded perspective views, FIG. 5 is a perspective view in which a needle fixation member 10 and a tube connection member 30 are separated from each other, and FIG. 6 is a cross-sectional view illustrating an internal configuration.

The needle infusion set 100 for insulin infusion according to the first embodiment of the present disclosure is a device that infuses insulin supplied from an insulin infusion device (not illustrated) into a body while inserting one infusion needle 21 into a body fat layer of the abdomen and includes a needle fixation member 10 and a tube connection member 30.

As illustrated in FIGS. 1 to 6, the needle fixation member 10 is configured to include a body 11, a base 12, a guide groove 13, an insertion groove 14, upper and lateral staking protrusions 15 and 16, a fitting protrusion 17, an upper insertion hole 18, a lateral insertion hole 19, an infusion needle insertion hole 20, an infusion needle 21, a cap insertion groove 22, a protection cap 23, and an attachment pad 50.

In the body 11 of the needle fixation member 10, the other side has a rounded curved shape, the body 11 is formed in a rectangular shape in which one side has a smaller width than the other side, and the insertion grooves 14 are formed on both lateral surfaces of the rectangle in a longitudinal direction. In this case, an insertion portion 32 of the tube connection member 30 is inserted into the insertion groove 14.

The body 11 has a base 12 which is flat with a step with the rectangular shape in both lateral surfaces and one lateral surface of the rectangle with the insertion groove 14.

In the body 11, the upper staking protrusion 15 is formed on an upper surface and the upper insertion hole 18 passing through an internal space is formed inside the upper staking protrusion 15.

Further, in the body 11, the lateral staking protrusion 16 is formed on one lateral surface and the lateral insertion hole 19 passing through the internal space is formed inside the lateral staking protrusion 16.

The upper insertion hole 18 and the lateral insertion hole 19 are connected to each other through the internal space of the body 11. The infusion needle insertion hole 20 is vertically formed in the internal space. The infusion needle insertion hole 20 may be formed in a funnel shape and is formed to penetrate through a lower surface of the body 11.

The infusion needle 21 is vertically installed in the infusion needle insertion hole 20. In this case, an upper end of the infusion needle 21 is positioned in the internal space and a lower end of the infusion needle 21 protrudes vertically from a lower portion of the body 11.

The infusion needle 21 may be vertically inserted into the infusion needle insertion hole 20 through the upper insertion hole 18 and then fixed with an adhesive and installed.

The infusion needle 21 preferably has a diameter of 31 to 33 gauges.

As one example, the infusion needle 21 is manufactured with a diameter of 31 gauge (diameter: 0.25 mm) and used for 20 patients. As a result, since the infusion needle is manufactured so that the diameter is smaller than 27 gauge (diameter: 0.47 mm) of the existing infusion needle, there is no pain when the infusion needle is inserted or removed into or from the skin, and the infusion needle flexibly moves with movement of the muscle even when the infusion needle is inserted into the skin, and as a result, there is no sense that the needle is inserted.

When the infusion needle 21 is installed in the infusion needle insertion hole 20, the upper sealing member 40 is inserted into the upper insertion hole 18 and seals the upper insertion hole 18 and then the upper staking protrusion 15 is staked to the inside of the upper insertion hole 18 to fix the upper sealing member 40.

Similarly, the lateral sealing member 41 is inserted into the lateral insertion hole 19 to seal the lateral insertion hole 19 and then the lateral staking protrusion 16 is staked to the inside of the lateral insertion hole 19 to fix the lateral sealing member 41.

Here, the upper and lateral staking protrusions 15 and 16 may be staked by heat staking. However, the upper and lateral staking protrusion may be staked by using ultrasonic waves or other means.

The upper and lateral sealing members 40 and 41 may be made of silicon rubber. Even though the infusion needle 37 is inserted and then removed, the lateral sealing member 41 is preferably made of a material sealed so as to prevent the insulin from being leaked.

For reference, an insulin infusion device (not illustrated) is used in order to infuse the insulin into the body to be suitable for a change in blood sugar of the patient. The insulin infusion device (also referred to as an 'insulin pump') is a device that automatically infuses a predetermined insulin amount every predetermined time by driving a piston while inserting the infusion needle into the body fat layer of the abdomen at all times.

When the infusion needle 21 is inserted into the needle fixation member 10, the protection cap 23 may be covered in order to protect the infusion needle 21 which protrudes from the lower portion of the needle fixation member 10. The protection cap 23 is inserted into the circular cap insertion groove 22 formed on the lower surface of the base 12 to protect the infusion needle 21.

Continuously, the base 12 is integrally formed at the lower portion of the body 11. The base 12 is formed with an area extended to a predetermined size with steps from both lateral surfaces (i.e., both lateral surfaces with the insertion grooves 14) of the rectangle of the body 11 and the other surface with the lateral staking protrusion 16. In addition, the fitting protrusions 17 of the base 12 are formed to protrude on both peripheral portions.

The fitting protrusion 17 may be formed by a triangular pillar (e.g., a right-angled triangular pillar). The fitting protrusion 17 is fastened to the receiving groove 34 of the tube connection member 30 to fix the needle fixation member 10 and the tube connection member 30.

The base 12 has a guide groove 13 formed at an upper portion of the base 12 at a side where the lateral staking protrusion 16 is formed. When the tube connection member 30 is inserted into the needle fixation member 10, the guide groove 13 is formed to guide a convex portion of the body 31 where the infusion needle 37 and the tube 36 of the tube connection member 30 are disposed.

Further, the base 12 has a circular cap insertion groove 22 on the periphery of the infusion needle insertion hole 20 formed on the lower surface and the protection caps 23 and 27 are inserted into the cap insertion groove 22 to protect the infusion needle 21.

Continuously, the body 11 is configured by bonding the attachment pad 50 to the lower surface. A part of the top surface of the attachment pad 50 is bonded and fixed to the lower surface of the body 11 and the attachment pad 50 is attached to the skin of the patient to fix the needle fixation member 10. The attachment pad 50 may be configured by a medical tape or an integrated adhesive pad and a release sheet 51 is attached in order to protect an attachment surface.

When a diabetic patent intends to infuse the insulin, the needle fixation member 10 removes the protection cap 90 and then puts the infusion needle 21 on a body fat of the abdomen. In this case, the needle fixation member 10 is attached and fixed to the skin by the attachment pad 50 of the lower surface.

A material of the needle fixation member 10 may be formed by using K-resin, polypropylene, or medical plastic.

Next, the tube connection member 30 coupled to the needle fixation member 10 will be described.

As illustrated in FIGS. 1 to 6, the tube connection member 30 is configured to include a body 31, an insertion portion 32, a hook lever 33, a receiving groove 34, a hook protrusion 35, a tube 36, and an infusion needle 37.

The tube 36 supplying the insulin is connected to one side of the body 31 of the tube connection member 30 and the infusion needle 37 inserted into the sealing member 41 is formed to protrude on the other side.

The hook lever 33 is formed on both outer portions of the body 31 to have elastic force. The hook protrusion 35 and the receiving groove 34 are formed on the outside of the hook lever 33. When the tube connection member 30 is inserted into the needle fixation member 10, the fitting protrusion 17 of the needle fixation member 10 is fastened to the receiving groove 34.

The insertion portion 32 is formed inside the body 31 and the insertion portion 32 is formed between the hook lever 33 and the infusion needle 37. When the tube connection member 30 is coupled to the needle fixation member 10, the insertion portion 32 is inserted into the insertion groove 14 of the needle fixation member 10.

The material of the tube connection member 30 may be formed by using polycarbonate, polypropylene, or medical plastic.

In the needle fixation member 10 according to the first embodiment of the present disclosure, when the insulin is dosed from the insulin pump through the tube 36 and the infusion needle 37 of the tube connection member 30, the insulin is transferred to the infusion needle insertion hole 20 through the lateral insertion hole 19 to supply the insulin into the body through the infusion needle 21.

### Second embodiment

FIGS. 7 to 13 are diagrams illustrating a needle infusion set for insulin infusion according to a second preferred embodiment of the present disclosure; FIG. 7 is a perspective view, FIGS. 8 to 10 are exploded perspective views, and FIGS. 11 to 13 are cross-sectional views illustrating an internal configuration.

The needle infusion set 100 for insulin infusion according to the second embodiment of the present disclosure is a device that infuses insulin supplied from an insulin infusion device (not illustrated) into a body through the other infusion needle even though an infusion needle at one portion is blocked while inserting two infusion needles 21 and 25 into a body fat layer of the abdomen and includes a needle fixation member 10 and a tube connection member 30.

The second embodiment of the present disclosure is configured differently from the first embodiment in that two infusion needles 21 and 25 are formed in the needle fixation member 10. In addition, since the tube connection member 30 has the same configuration as the first embodiment, the tube connection member 300 will be omitted herein.

As illustrated in FIGS. 7 to 13, the needle fixation member 10 is configured to include a body 11, a base 12, a guide groove 13, an insertion groove 14, upper and lateral staking protrusions 15 and 16, a fitting protrusion 17, an upper insertion hole 18, a lateral insertion hole 19, infusion needle insertion holes 20 and 24, infusion needles 21 and 27, cap insertion grooves 22 and 26, protection caps 23 and 27, and an attachment pad 50.

In the body 11, the upper staking protrusion 15 is formed on an upper surface and the upper insertion hole 18 passing through an internal space is formed inside the upper staking protrusion 15. In this case, the upper staking protrusion 15 and the upper insertion hole 18 have a shape in which two ellipses are connected to each other and have a size twice larger than that of the first embodiment.

Further, in the body 11, the lateral staking protrusion 16 is formed on one lateral surface and the lateral insertion hole 19 passing through the internal space is formed inside the lateral staking protrusion 16. The lateral staking protrusion 16 and the lateral insertion hole 19 are the same as those of the first embodiment.

The upper insertion hole 18 and the lateral insertion hole 19 are connected to each other through the internal space of the body 11 (see FIGS. 11 to 13).

The two infusion needle insertion holes 20 and 24 are vertically formed at an internal lower end of the upper insertion hole 18. The infusion needle insertion holes 20 and 24 are formed in a funnel shape and formed to penetrate through the lower surface of the body 11.

The lateral insertion holes 19 are formed in the upper internal space of the infusion needle insertion hole 24 and connected to each other through a connection hole 28 between the infusion needle insertion holes 20 and 24.

The infusion needles 21 and 25 are vertically installed in the infusion needle insertion holes 20 and 24. In this case, upper ends of the infusion needles 21 and 25 are positioned in the internal space and lower ends of the infusion needles 21 and 25 protrude vertically from the lower portion of the body 11.

The infusion needles 21 and 25 may be vertically inserted into the infusion needle insertion holes 20 and 24 through the upper insertion hole 18 and then fixed with an adhesive and installed.

When the infusion needles 21 and 25 are installed in the infusion needle insertion holes 20 and 24, the upper sealing member 40 is inserted into the upper insertion hole 18 and seals the upper insertion hole 18 and then the upper staking protrusion 15 is staked to the inside of the upper insertion hole 18 to fix the upper sealing member 40.

Similarly, the lateral sealing member 41 is inserted into the lateral insertion hole 19 to seal the lateral insertion hole 19 and then the lateral staking protrusion 16 is staked to the inside of the lateral insertion hole 19 to fix the lateral sealing member 41.

Here, the upper and lateral staking protrusions 15 and 16 may be staked by heat staking. However, the upper and lateral staking protrusions 15 and 16 may be staked by using ultrasonic waves or other means.

The upper and lateral sealing members 40 and 41 may be made of silicon rubber. Further, the upper and lateral sealing members 40 and 41 may be configured by using polyurethane or other rubber materials. Even though the infusion needle 37 is inserted and then removed, the lateral sealing member 41 is preferably made of a material sealed so as to prevent the insulin from being leaked.

In the needle fixation member 10 according to the second embodiment of the present disclosure, when the insulin is dosed from the insulin pump through the tube (see reference numeral 36 of FIG. 5) and the infusion needle 37 of the tube connection member 30, the insulin is transferred to the infusion needle insertion hole 20 through the lateral insertion hole 19 and the insulin is simultaneously transferred to the infusion needle insertion hole 20 through the connection hole 28 to supply the insulin into the body through two infusion needles 21 and 25.

Meanwhile, in the second embodiment of the present disclosure, an example is described in which two infusion needles 21 and 25 are configured in the needle fixation member 10, but a plurality of two or more infusion needles may be configured.

Furthermore, as in the second embodiment of the present disclosure, two or more multi-channel infusion needles are integrally configured to reduce crumpling of a subcutaneous fat layer which frequently occurs in insulin infusion due to dispersion of an insulin liquid.

As described above, the needle infusion set according to the present disclosure provides a fluid path in which the insulin pumped from the insulin infusion device (insulin pump) is transferred to one or two or more of a plurality of infusion needles through the tube connection member and the needle fixation member and infused into the body, thereby solving the technical problem.

Preferred embodiments of the present disclosure described above are disclosed to solve the technical problem and various modifications, changes, additions etc., will be able to be made within the scope of the present disclosure by those skilled in the art and it should be appreciated that the modification and changes belong to the following claims.

## Claims

1. A needle infusion set for insulin infusion, comprising:
a needle fixation member (10); and
a tube connection member (30),
wherein the needle fixation member (10) includes:
a body (11) in which one side is configured in an elliptical shape and an other side is configured in a rectangular shape wherein the other side has a smaller width than the one side, insertion grooves (14) are formed on both lateral surfaces of the other side which is configured in the rectangular shape, a base (12) is formed to be flat with steps from the other side which is configured in the rectangular shape, a guide groove (13) is formed at one upper portion of the base (12) in a longitudinal direction, and fitting protrusions (17) are vertically formed on both lateral surfaces of the base (12),
upper and lateral staking protrusions (15, 16) formed to protrude from a top surface of the body (11) and a lateral surface of the body (11) and having upper and lateral insertion holes (18, 19) passing through an internal space therein,
two or more infusion needle insertion holes (20, 24) formed to vertically penetrate through a lower surface of the body in the internal space of the body (11).
two or more infusion needles (21, 25) vertically installed in the infusion needle insertion holes (20, 24) and fixed with an adhesive,
upper and lateral sealing members (40, 41) inserted into the upper and lateral insertion holes (18, 19) and sealing the internal space and then fixed by staking the upper and lateral staking protrusions (15, 16), and
protection caps (23, 27) inserted into cap insertion grooves (22, 26) formed on the lower surface of the body (11) and protecting the infusion needles (21, 25),
wherein the needle infusion set for insulin infusion simultaneously supplies the insulin through a connection hole (28) connecting the infusion needle insertion holes (20, 24) to each other inside the body (11) when there are two or more infusion needle insertion holes (20, 24).

2. The needle infusion set for insulin infusion of claim 1, wherein the tube connection member (30) includes
a body (31) in which a tube (36) supplying insulin is connected to one side of the body (31) and an infusion needle (37) inserted into the lateral sealing member (41) protrudes on an other side of the body (31),
a hook lever (33) formed to have elastic force on both outer sides of the body (31) and having receiving grooves (33) to which a hook protrusion (35) and the fitting protrusion (17) are fastened at an outer side, and
an insertion portion (32) formed between the hook lever (33) and the infusion needle (37) and inserted into the insertion groove (14).

3. The needle infusion set for insulin infusion of claim 1, wherein the needle fixation member (10) further includes an attachment pad (50) bonded to the lower surface of the body (11) and attached to the body to fix the needle fixation member (10).

4. The needle infusion set for insulin infusion of claim 1, wherein the infusion needles (21, 25) have a diameter of 31 to 33 gauges.

## Patentansprüche

1. Nadelinfusionsset für Insulininfusion, umfassend:
ein Nadelfixierungselement (10); und
ein Rohrverbindungselement (30),
wobei das Nadelfixierungselement (10) Folgendes beinhaltet:
einen Körper (11), bei dem eine Seite in einer elliptischen Form konfiguriert ist und eine andere Seite in einer rechteckigen Form konfiguriert ist, wobei die andere Seite eine kleinere Breite als die eine Seite aufweist, Einführungsnuten (14) an beiden lateralen Oberflächen der anderen Seite gebildet sind, die in der rechteckigen Form konfiguriert ist, eine Basis (12) gebildet ist, um flach mit Stufen von der anderen Seite zu sein, die in der rechteckigen Form konfiguriert ist, eine Führungsnut (13) an einem oberen Abschnitt der Basis (12) in einer Längsrichtung gebildet ist und Passvorsprünge (17) vertikal an beiden lateralen Oberflächen der Basis (12) gebildet sind,
obere und laterale Verstemmvorsprünge (15, 16), die gebildet sind, um von einer oberen Oberfläche des Körpers (11) und einer lateralen Oberfläche des Körpers (11) vorzuspringen, und obere und laterale Einführungslöcher (18, 19) aufweisen, die durch einen Innenraum darin verlaufen,
zwei oder mehr Infusionsnadeleinführungslöcher (20, 24), die gebildet sind, um vertikal durch eine untere Oberfläche des Körpers in den Innenraum des Körpers (11) einzudringen,
zwei oder mehr Infusionsnadeln (21, 25), die vertikal in den Infusionsnadeleinführungslöchern (20, 24) installiert und mit einem Klebstoff fixiert sind,
obere und laterale Dichtungselemente (40, 41), die in die oberen und lateralen Einführungslöcher (18, 19) eingeführt sind und den Innenraum abdichten und dann durch Verstemmen der oberen und lateralen Verstemmvorsprünge (15, 16) fixiert sind, und
Schutzkappen (23, 27), die in Kappeneinführungsnuten (22, 26) eingeführt sind, die an der unteren Oberfläche des Körpers (11) gebildet sind und die Infusionsnadeln (21, 25) schützen,
wobei das Nadelinfusionsset für Insulininfusion gleichzeitig das Insulin durch ein Verbindungsloch (28) zuführt, das die Infusionsnadeleinführungslöcher (20, 24) innerhalb des Körpers (11) miteinander verbindet, wenn es zwei oder mehr Infusionsnadeleinführungslöcher (20, 24) gibt.

2. Nadelinfusionsset für Insulininfusion nach Anspruch 1, wobei das Rohrverbindungselement (30) einen Körper (31), in dem ein Rohr (36), das Insulin zuführt, mit einer Seite des Körpers (31) verbunden ist und eine Infusionsnadel (37), die in das laterale Dichtungselement (41) eingeführt ist, auf einer anderen Seite des Körpers (31) vorspringt, einen Hakenhebel (33), der gebildet ist, um elastische Kraft an beiden Außenseiten des Körpers (31) aufzuweisen und Aufnahmenuten (33) aufweist, an denen ein Hakenvorsprung (35) und der Passvorsprung (17) an einer Außenseite befestigt sind, und einen Einführungsabschnitt (32), der zwischen dem Hakenhebel (33) und der Infusionsnadel (37) gebildet und in die Einführungsnut (14) eingeführt ist, beinhaltet.

3. Nadelinfusionsset für Insulininfusion nach Anspruch 1, wobei das Nadelfixierungselement (10) ferner ein Anbringungspolster (50) beinhaltet, das an die untere Oberfläche des Körpers (11) gebunden und an dem Körper angebracht ist, um das Nadelfixierungselement (10) zu fixieren.

4. Nadelinfusionsset für Insulininfusion nach Anspruch 1, wobei die Infusionsnadeln (21, 25) einen Durchmesser von 31 bis 33 Gauge aufweisen.

## Revendications

1. Ensemble de perfusion à aiguille pour perfusion d'insuline, comprenant :
un élément de fixation d'aiguille (10) ; et
un élément de raccordement de tube (30),
dans lequel l'élément de fixation d'aiguille (10) comprend :
un corps (11) dans lequel un côté est conçu en une forme elliptique et un autre côté est conçu en une forme rectangulaire, dans lequel l'autre côté présente une largeur plus petite que le premier côté, des rainures d'insertion (14) sont formées sur les deux surfaces latérales de l'autre côté qui est conçu en forme rectangulaire, une base (12) est formée pour être plate avec des marches à partir de l'autre côté qui est conçu en forme rectangulaire, une rainure de guidage (13) est formée au niveau d'une partie supérieure de la base (12) dans une direction longitudinale, et des saillies d'ajustement (17) sont formées verticalement sur les deux surfaces latérales de la base (12),
des saillies d'implantation supérieure et latérale (15, 16) formées pour faire saillie à partir d'une surface supérieure du corps (11) et d'une surface latérale du corps (11) et présentant des trous d'insertion supérieur et latéral (18, 19) traversant un espace interne à l'intérieur,
deux trous d'insertion d'aiguille de perfusion (20, 24) ou plus formés pour pénétrer verticalement à travers une surface inférieure du corps dans l'espace interne du corps (11)
deux aiguilles de perfusion (21, 25) ou plus installées verticalement dans les trous d'insertion d'aiguille de perfusion (20, 24) et fixées avec un adhésif,
des éléments d'étanchéité supérieur et latéral (40, 41) insérés dans les trous d'insertion supérieur et latéral (18, 19) et scellant l'espace interne, puis fixés en piquetant les saillies d'implantation supérieure et latérale (15, 16), et
des capuchons de protection (23, 27) insérés dans des rainures d'insertion de capuchon (22, 26) formées sur la surface inférieure du corps (11) et protégeant les aiguilles de perfusion (21, 25),
dans lequel l'ensemble de perfusion à aiguille pour perfusion d'insuline fournit simultanément l'insuline à travers un trou de raccordement (28) raccordant les trous d'insertion d'aiguille de perfusion (20, 24) les uns aux autres à l'intérieur du corps (11) lorsqu'il y a deux trous d'insertion d'aiguille de perfusion (20, 24) ou plus.

2. Ensemble de perfusion à aiguille pour perfusion d'insuline de la revendication 1, dans lequel l'élément de raccordement de tube (30) comprend un corps (31) où un tube (36) fournissant de l'insuline est raccordé à un côté du corps (31) et une aiguille de perfusion (37) insérée dans l'élément d'étanchéité latéral (41) fait saillie d'un autre côté du corps (31),
un levier à crochet (33) formé pour présenter une force élastique sur les deux côtés extérieurs du corps (31) et présentant des rainures de réception (33) auxquelles une saillie de crochet (35) et la saillie d'ajustement (17) sont fixées sur un côté extérieur, et
une partie d'insertion (32) formée entre le levier à crochet (33) et l'aiguille de perfusion (37) et insérée dans la rainure d'insertion (14).

3. Ensemble de perfusion à aiguille pour perfusion d'insuline de la revendication 1, dans lequel l'élément de fixation d'aiguille (10) comprend en outre un tampon d'attache (50) lié à la surface inférieure du corps (11) et attaché au corps pour fixer l'élément de fixation d'aiguille (10).

4. Ensemble de perfusion à aiguille pour perfusion d'insuline de la revendication 1, dans lequel les aiguilles de perfusion (21, 25) présentent un diamètre de 31 à 33 gauges.
